# EUROPÄISCHE PATENTANMELDUNG

(11) **EP 1 586 317 A1**
(43) Veröffentlichungstag der Anmeldung: **19.10.2005**
(21) Anmeldenummer: 05450039.2
(22) Anmeldetag: 03.03.2005
(51) Int. Cl.: A61K 31/205, A61K 31/385, A61K 31/522, A61K 31/685, A61P 43/00

(54) **Orale Zusammensetzung L-Carnitin und ein Xantihin**

(30) Priorität: 05.03.2004 AT 3912004
(71) Anmelder: Lohninger, Alfred, 2531 Gaaden (AT)
(72) Erfinder: Lohninger, Alfred, 2531 Gaaden (AT)
(74) Vertreter: Haffner, Thomas M.

(57) **Zusammenfassung**

Bei einer oral verabreichbaren Zusammensetzung zur Verbesserung der Energiegewinnung aus Fettsäuren im oxidativen Abbau enthaltend L-Carnitin sind L-Carnitin und/oder dessen wasserlösliche Salze bzw. C₂- oder C₃-Ester gemeinsam mit Mono-, Di- oder Trimethylxanthinen sowie veresterten essentiellen Fettsäuren in Form von essentiellen Phospholipiden sowie Liponsäure enthalten.

## Beschreibung

Die Erfindung bezieht sich auf eine oral verabreichbare Zusammensetzung zur Verbesserung der Energiegewinnung aus Fettsäuren im oxidativen Abbau enthaltend L-Carnitin.

Aus der EP 1 014 965 B1 ist eine oral verabreichbare trockene Einheitsdosierung bekannt geworden, welche Carnitin und ein mitochondrial aktives Antioxidans, welches physiologisch eine metabolisch reaktive Thiolgruppe umfasst, enthält. Derartige Darreichungsformen bzw. Einheitsdosierungen erlauben es, den Metabolismus zu steigern und oxidativen Stress zu lindern. Prinzipiell sind derartige Produkte als Nahrungsergänzung geeignet und verlangsamen den biologisch bedingten Alterungsprozeß. Gleichzeitig werden natürliche Abwehrkräfte gestärkt, die Merk-, Lern- und Leistungsfähigkeit des Gehirns verbessert und der oxidative Stoffwechsel verjüngt. Unter Verjüngung des oxidativen Stoffwechsels versteht man hiebei, dass vermehrt Fettsäuren zur Energiegewinnung im oxidativen Stoffwechsel verwertet werden, was dem Herz- und Skelettmuskeln eine erhöhte Energieproduktion ermöglicht.

Unausgewogene Ernährung, die Verringerung des Nährstoffgehaltes bei industriell gefertigten Nahrungsmitteln, hohe Umwelt- und Stressbelastung, hoher Kaffee-, Tabak-, Alkohol- und Arzneimittelkonsum, biologisch bedingte Alterungsprozeße und vieles andere mehr belasten den Organismus und beeinträchtigen das Wohlbefinden und die Gesundheit. Da die meisten dieser Belastungen mit einer vermehrten Bildung von Radikalen verbunden sind, wurde bereits vorgeschlagen zur Verlangsamung des biologisch bedingten Alterungsprozeßes Radikalfänger bzw. Antioxidantien zuzusetzen. Wenn der oxidative Stoffwechsel stimuliert wird, ist es in der Regel nicht vermeidbar, dass aus rund 2% des ungesetzten Sauerstoffs Sauerstoffradikale entstehen. Der Zusatz von Antioxidantien erlaubt es somit diese zusätzlich entstehenden Sauerstoffradikale abzufangen.

Für die Wirksamkeit der den oxidativen Abbau stimulierenden Substanzen, und insbesondere von Carnitin, ist aber vor allem eine entsprechende intrazelluläre Konzentration notwendig. Wenn der Transport von Carnitin in die Zelle durch die Zellmembran nicht hinreichend gelingt, ist auch der Zusatz von Carnitin in nahrungsergänzenden Zusammensetzungen weitestgehend wirkungslos. Carnitin kann im Darm bakteriell abgebaut werden bevor es wirksam werden kann.

Für die Carnitinaufnahme wurden Na+ abhängige und Na+ unabhängige Transportsysteme aufgefunden, die sich auch in ihrer Affinität unterscheiden. Die intrazelluläre Konzentration des Carnitins wird hiebei durch verschiedene Membrantransporter kontrolliert, wobei der humane Kationentransporter OCTN2 physiologisch besonders wichtig ist. Die Produktion von OCTN2 nimmt mit zunehmendem Alter und bei hohen Konzentrationen an Sauerstoffradikalen ab.

Die Erfindung zielt nun darauf ab, die intrazelluläre Konzentration von Carnitin erhöhenden oral verabreichbaren Zusammensetzungen, und insbesondere nahrungsergänzenden Zusammensetzungen, zu verbessern, um auf diese Weise die eingangs beschriebenen, erwünschten Vorteile zu erzielen.

Zur Lösung dieser Aufgabe besteht die erfindungsgemäße oral verabreichbare Zusammensetzung enthaltend L-Carnitin im Wesentliche darin, dass L-Carnitin und/oder dessen wasserlösliche Salze bzw. C₂- oder C₃-Ester gemeinsam mit Mono-, Di- oder Trimethylxanthinen sowie veresterten essentiellen Fettsäuren in Form von essentiellen Phospholipiden sowie Liponsäure enthalten sind. Da Xanthine und essentielle Phospholipide gemeinsam mit L-Carnitin eingesetzt werden, wird die OCTN2-Produktion erhöht und damit die Wirksamkeit von Carnitin beim oxidativen Fettabbau durch Verbesserung des Transports durch die Zellmembran und damit durch Erhöhung der intrazellulären Konzentration von Carnitin deutlich verbessert. Insbesondere konnte in tierexperimentellen Studien und Untersuchungen an freiwilligen Probanden gezeigt werden, dass die regelmäßige Zufuhr oder Einnahme derartiger oral verabreichbarer Zusammensetzungen die Sauerstoffaufnahme durch wichtige Gewebe, wie Gehirn, Niere -und -Leber, vermehrt und gleichzeitig die Spiegel an Oxidationsprodukten von Lipiden nach Lipidperoxidation, z.B. Malondialdehyd, vermindert. Von wesentlicher Bedeutung ist hiebei der Umstand, dass die intrazelluläre Konzentration von Carnitin ein entscheidender Kontrollfaktor der Transkription von zahlreichen Genen des oxidativen Stoffwechsels ist. Der mRNA-Gehalt dieser Enzyme ist bei niedrigem Carnitinspiegel stark vermindert und kann durch Carnitinsubstitution erhöht werden, wobei durch die gleichzeitige Stimulierung des OCTN2 mehr als nur additive Effekte erreichbar sind. Die erhöhte Transkription (mRNA) von Schlüsselenzymen der Fettsäurenoxidation, und insbesondere der Carnitin-Palmitoyltransferase 1 (CPT1A) und besonders der muskelständigen Isoform (CPT1B), der mikrosomalen Isoform von CPT1A, sowie der Carnitin-Acetyltransferase, führt zu der gewünschten Verbesserung des oxidativen Abbaues. Gleichzeitig steigt der Cardiolipingehalt im Gewebe auf das Niveau jüngerer Gewebe an und die intrazellulären Spiegeln an Carnitin steigen auf ein Niveau, welches mit alleiniger Carnitinzufuhr nicht erreicht werden kann. Der Liponsäurezusatz führt zu einer Hemmung der Fettzellendifferenzierung und ist unter anderem auch als Antioxidans wirksam.

Als besonders bevorzugt hat sich für die Stimulierung der OCTN2-Produktion das Dimethylxanthin, nämlich Theophyllin, erwiesen.

Die Zugabe von essentiellen Phospholipiden dient gleichfalls der Verbesserung des Transports durch die Zellmembran, wobei hier bevorzugt als essentielle Phospholipide Phosphatidylcholin (Lecithin) oder Phosphatidylglycerol, insbesondere jene Spezies die mit Linol- und/oder Linolensäure verestert sind, eingesetzt sind.

Zusätzlich zu den erfindungsgemäß vorgeschlagenen Maßnahmen zur Erhöhung der intrazellulären Carnitinkonzentration kann aber auch, wie an sich bekannt, noch dem Umstand Rechnung getragen werden, dass ein stimulierter Fettsäureabbau gleichzeitig zu einer Zunahme von Radikalen führt. Es können daher zusätzlich noch bekannte Antioxidantien, und insbesondere bevorzugt Antioxidantien aus der Gruppe der Flavonoide, β-Carotinoide und/oder Vitamine C und/oder E, eingesetzt werden. In an sich bekannter Weise kann die Zusammensetzung auch Zinksalze oder -oxide enthalten.

Für die Hemmung der Fettzellendifferenzierung können weitere Substanzen, wie beispielsweise Liponsäure oder L-Cystein eingesetzt werden. Derartige Verbindungen müssen aber in aller Regel verkapselt eingesetzt werden, da sie geschmacklich in Nahrungsmitteln nur geringe Akzeptanz finden würden. Während Liponsäure im Wesentlichen pfeffrig und scharf schmeckt und aus diesem Grund einer Verkapselung bedarf, müssen auch die erfindungsgemäß vorgeschlagenen Xanthine auf Grund ihres gallbitteren Geschmackes mikroverkapselt werden, um in Nahrungsergänzungsmitteln akzeptiert zu werden.

Die erfindungsgemäßen oral verabreichbaren Zusammensetzungen können unmittelbar Nahrungsmitteln zugesetzt werden, wobei auf die maximal wünschenswerten Tagesdosen Rücksicht genommen werden muss. Bei einer Vorabzusammensetzung, welche 10 bis 10.000mg L-Carnitintartrat enthält, reichen 1 bis 1.000mg Xanthine, 0,05 bis 1 Gewichtsteil Vitamin A, 5 bis 100 Gewichtsteile Vitamin E und 1 bis 5 Gewichtsteile Zink bezogen auf den Zinkgehalt des Salzes oder Oxid aus, um ein leicht dosierbares Vorprodukt zu erhalten, welches in der Folge so zugesetzt wird, dass die maximale Xanthinmenge beispielsweise etwa 100mg/Tag nicht überschreitet.

Bioflavonoide können als Extrakt der roten Traubenschalen eingesetzt werden, wobei die in Nahrungsergänzungsmitteln bereits weitestgehend üblichen Zusätze von Vitaminen A,E und C in den üblichen Grenzen gehalten werden. Dies bedeutet insbesondere, dass die Verhältnisse von Vitamin A,E und C üblicher Weise 0,05 bis 0,1 Gewichtsteile Vitamin A, 7 bis 10 Gewichtsteile Vitamin E und 40 bis 80 Gewichtsteile Vitamin C enthalten.

## Patentansprüche

1. Oral verabreichbare Zusammensetzung zur Verbesserung der Energiegewinnung aus Fettsäuren im oxidativen Abbau enthaltend L-Carnitin, **dadurch gekennzeichnet, dass** L-Carnitin und/oder dessen wasserlösliche Salze bzw. C₂- oder C₃-Ester gemeinsam mit Mono-, Di- oder Trimethylxanthinen, veresterten essentiellen Fettsäuren in Form von essentiellen Phospholipiden sowie Liponsäure enthalten sind.

2. Oral verabreichbare Zusammensetzung nach Anspruch 1, **dadurch gekennzeichnet, dass** als Xanthinderivat Theophyllin eingesetzt ist.

3. Oral verabreichbare Zusammensetzung nach Anspruch 1 oder 2, **dadurch gekennzeichnet, dass** als essentielle Phospholipide Phosphatidylcholin (Lecithin) oder Phosphatidylglycerol, insbesondere jene Spezies die mit Linol- und/oder Linolensäure verestert sind, eingesetzt sind.

4. Oral verabreichbare Zusammensetzung nach Anspruch 1,2 oder 3, **dadurch gekennzeichnet, dass** die Zusammensetzung zusätzlich Antioxidantien aus der Gruppe der Flavonoide, β-Carotinoide und/oder Vitamine C und/oder E enthält.

5. Oral verabreichbare Zusammensetzung nach einem der Ansprüche 1 bis 4, **dadurch gekennzeichnet, dass** die Liponsäure in microverkapselter Form eingesetzt ist.

6. Oral verabreichbare Zusammensetzung nach einem der Ansprüche 1 bis 5, **dadurch gekennzeichnet, dass** die Xanthine in microverkapselter Form eingesetzt sind.
